# EUROPÄISCHE PATENTANMELDUNG

(11) **EP 2 962 734 A1**
(43) Veröffentlichungstag der Anmeldung: **06.01.2016**
(21) Anmeldenummer: 15175213.6
(22) Anmeldetag: 03.07.2015
(51) Int. Cl.: A61Q 19/10, B65D 75/50

(54) **DOSIERVORRICHTUNG**

(30) Priorität: 03.07.2014 DE 102014109313
(71) Anmelder: IdeaPRO GmbH, 68159 Mannheim (DE)
(72) Erfinder: Baumgärtner, Markus, 68199 Mannheim (DE); Zeilfelder, Florian, 68163 Mannheim (DE)
(74) Vertreter: Thews, Karl

(57) **Zusammenfassung**

Die Erfindung bezieht sich auf ein System bestehend aus einem als Formkörper gebildeten, kosmetischen Badezusatz 7 und einer Dosiervorrichtung 1, wobei der Badezusatz 7 in der Dosiervorrichtung 1 enthalten ist und bei Kontakt mit Wasser 9.1 unter Erzeugung eines Gases chemisch reagiert. Die Dosiervorrichtung 1 ist durch ein Behälter 2 mit einer festen und/oder flexiblen Behälterwand 2.1 gebildet, wobei der Behälter 2 eine rechtwinklig zu einer Achse X angeordnete Standfläche 3 und ein in Richtung der Achse X gegenüberliegend zur Standfläche 3 angeordnetes und entfernbares Verschlussteil 4 einer Öffnung 5 aufweist. Das Sprudeln des Badezusatzes 7 soll länger andauern und gleichzeitig soll eine sichere Handhabung gewährleistet sein. Hierzu ist der Badezusatz 7 als Formkörper gebildet und die Größe des Formkörpers und/oder die Größe der Öffnung 5 sind so gewählt, dass der Formkörper nicht durch die Öffnung 5 hindurch passt, wobei der Behälter 2 wasserunlöslich ist.

## Beschreibung

Die Erfindung bezieht sich auf ein System bestehend aus einem als Formkörper gebildeten, kosmetischen Badezusatz und einer Dosiervorrichtung, wobei der Badezusatz in der Dosiervorrichtung enthalten ist und bei Kontakt mit Wasser unter Erzeugung eines Gases chemisch reagiert. Die Dosiervorrichtung ist durch einen Behälter mit einer zumindest teilweise flexiblen Behälterwand gebildet, wobei der Behälter ein um eine Achse X angeordnetes und entfernbares Verschlussteil für zumindest eine Öffnung aufweist.

Es ist bereits ein Badezusatz in Form von Kugeln aus der US 2014/0079896 A1 bekannt, der im Wasser sprudelt. Diese verschiedenfarbigen Badekugeln sind in einer Verpackung in Form einer durchsichtigen Flasche verpackt und werden aus der Flasche heraus in das Wasser gegeben.

Nach der DE 602 25 462 T2 sind Badezusätze bekannt, die in wasserlöslichen Verpackungen enthalten sind. Diese Verpackungen werden beispielsweise auch in Form von Tetraedern mit dem Badezusatz in das Wasser gegeben. Nach dem zumindest teilweise Auflösen der Verpackung verteilt sich der Badezusatz im Wasser und löst sich ebenfalls auf.

Der Erfindung liegt die Aufgabe zugrunde einen Badezusatz derart auszubilden und anzuordnen, dass das Sprudeln länger andauert und gleichzeitig, besonders für Kinder, eine sichere Handhabung gewährleistet ist.

Gelöst wird die Aufgabe erfindungsgemäß dadurch, dass die Größe des Formkörpers und/oder die Größe der Öffnung so gewählt ist, dass der Formkörper nicht durch die Öffnung hindurch passt, wobei der Behälter wasserunlöslich ist.

Hierdurch wird erfindungsgemäß erreicht, dass der Badezusatz nur zusammen mit dem Behälter in das Wasser gegeben werden kann. Obwohl das gesamte System in das Wasser gegeben wird, ist der Behälter nicht wasserlöslich, damit durch den Behälter die Reaktion des Badezusatzes mit Wasser verzögert wird. Gleichzeitig ist es nicht möglich, den sich sprudelnd auflösenden Badezusatz direkt in die Hand zu nehmen, was die Sicherheit für Kinder erhöht. Das Wasser muss zunächst durch die Öffnung in den Behälter eindringen und der Wasserzulauf ist durch das gleichzeitig ausströmende Gas begrenzt. Dadurch kommt der Badezusatz im Verhältnis zu der maximal möglichen Umsetzungsrate von Gas nur mit sehr wenig Wasser in Berührung. Die Sprudeldauer erhöht sich dadurch. Ferner bleibt der sich teilweise zu sandförmiger Konsistenz auflösende Badezusatz in der Dosiervorrichtung und verteilt sich nicht im Badegefäß. Unangenehme Krümel am Gefäßboden oder ein direkter Kontakt mit der Haut werden dadurch vermieden und das System beziehungsweise die Dosiervorrichtung kann während des Sprudelvorgangs angefasst werden, ohne dass die Kugel oder die Tablette zerfällt.

Die Größe der Öffnung weist dabei ein Maß auf, durch das gewährleistet ist, dass der entstehende Gasstrom groß genug ist, damit sich die Dosiervorrichtung nicht vollständig mit Wasser füllt. Nach dem Eintauchen der geöffneten Dosiervorrichtung in das Wasser dringt Wasser in den Behälter ein und gleichzeitig entweicht die im Behälter enthaltene Luft. Mit der sofort einsetzenden Reaktion des Badezusatzes entsteht ein Volumen an Gas, das wesentlich größer ist, als das Volumen an Wasser, das für ein bestimmtes Maß einer Reaktion benötigt wird. Es soll nicht zu wenig, aber auch nicht zu viel sprudeln, damit einerseits ein Sprudeleffekt erzielt wird, aber andererseits der Sprudelvorgang eine gewisse Zeit andauert. Das austretende Gas verhindert bis zu einer gewissen Größe der Öffnung, dass sich der Behälter vollständig mit Wasser füllt. Würde die Öffnung zu groß gewählt, würde sich der gesamte Behälter mit Wasser füllen, eine heftige Reaktion auslösen und die Reaktionszeit wäre zu kurz.

Nach dem Entfernen des Verschlussteils können auch mehrere Öffnungen gegeben sein, beispielsweise in Form eines Siebes oder Gitters. Wesentlich für die Erfindung ist, dass jede Öffnung für sich eine Größe nicht überschreitet, durch die der Badezusatz aus dem Behälter herausfallen könnte.

Bevorzugt ist es, wenn ein Ende des Behälters in Richtung der Achse X eine Spitze formt, wobei die Spitze das Verschlussteil bildet und durch das Entfernen der Spitze die Öffnung entsteht. Das Entfernen der Spitze durch Abschneiden oder Abreißen erzeugt eine einfache Öffnung des Behälters, die im Wasser aufgrund des Austritts des Gases automatisch nach oben gerichtet ist. Das Verschlussteil ist in dieser Ausgestaltung ein Einwegteil, weil der Behälter nicht wieder damit verschlossen werden kann. Durch eine der Öffnung gegenüberliegende Standfläche wird verhindert, dass der Badezusatz aus der Öffnung herausfällt oder beim Sprudel herausquillt, während der Behälter im Wasser schwimmt oder auf dem Grund des Wassers aufliegt. Die Dosiervorrichtung liegt ohnehin aufgrund ihrer im Vergleich zu Wasser größeren Dichte zumindest in den ersten Minuten auf dem Boden des Badegefäßes auf der Standfläche auf. Durch die durch die Öffnung austretenden Gasblasen wird die Dosiervorrichtung stabilisiert. Unterschreitet der Badezusatz während des Sprudelns eine gewisse Menge, beginnt das System aufgrund der Gewichtsabnahme des Badezusatzes zu schwimmen.

Vorteilhaft kann es hierzu auch sein, wenn die Behälterwand im Bereich der Spitze um die Achse X umlaufend in einem Winkel a von mindestens 10° [Grad] relativ zur Achse X angeordnet ist. Durch die Eigenschaft des Materials der Behälterwand und durch die Geometrie der Spitze wird ein Verkleben der Öffnung verhindert. Die Behälterwand weist dadurch nach dem Entfernen der Spitze ausreichend Elastizität auf, um die Öffnung aufzuspannen.

Hinsichtlich einer stabilen Sprudelreaktion im Behälter kann es vorteilhaft sein, wenn der Behälter die Form eines Tetraeders aufweist und das Verschlussteil in Richtung der Achse X gegenüberliegend zur Standfläche angeordnet ist. In diesem Fall ist die Standfläche gegenüber der Öffnung ausgebildet. Mit einer Tetraederform kann eine sehr hohe Stabilität unter Wasser erreicht werden. Der sich ausgehend von der Standfläche kontinuierlich nach oben hin verjüngende und spitz zulaufende Behälter wird durch die nach dem Öffnen austretenden Gase automatisch auf der Standfläche stabilisiert.

Alternativ kann es von Vorteil sein, wenn der Behälter in Form eines Zylinders mit einem Verhältnis 10:1 ausgebildet ist, nämlich der Länge in Richtung der Achse X im Verhältnis zum Durchmesser in einer Richtung rechtwinklig zur Achse X. Ein solcher zylinderförmiger Stab hat den Vorteil, dass er als Rührer zum Mischen des Badezusatzes mit dem Wasser eingesetzt werden kann.

Vorteilhaft kann es auch sein, wenn der Badezusatz in Form von Tabletten oder Kugeln ausgebildet ist, wobei der mittlere Durchmesser der Tabletten oder der Kugeln zwischen 4 mm und 15 mm beträgt. Ein solches schüttfähiges Gefüge des Badezusatzes lässt sich in jeder Form in einen Behälter einfüllen. Im Vergleich zu einer Badekugel weist ein solches Gefüge bei gleicher Masse und bei gleichem Volumen eine sehr viel größere Oberfläche auf, wodurch ein größerer Sprudeleffekt entsteht.

Dabei kann es vorteilhafterweise vorgesehen sein, dass der Behälter aus einem Verbundstoff gebildet ist, der eine gas- und feuchtigkeitsdichte Innenschicht und eine auf die Innenschicht mittel- oder unmittelbar aufgebrachte Außenschicht aufweist, wobei die Außenschicht aus einem Trägermaterial und/oder aus einer Armierung gebildet ist. Der Badezusatz wird hierdurch bei verschlossenem Behälter trocken gehalten, so dass keine Luftfeuchtigkeit zu einer zwar geringen aber dennoch frühzeitigen Reaktion führen kann. Solche Materialien für Behälter oder Behälterwände können durch Falten und/oder Biegen sowie Kleben und/oder Schweißen aus einem bahnenförmigen Material hergestellt werden.

Von besonderer Bedeutung kann für die vorliegende Erfindung sein, wenn der Behälter um die Achse X umlaufend unterhalb der Spitze eine Markierung, teilweise einen Schlitz und/oder eine Sollreißlinie aufweist, welche einen Rand für die Öffnung definiert. Die Größe der Öffnung ist maßgeblich für die Dauer und die Intensität der Reaktion, so dass je nach Badezusatz ein im Vorfeld definierter Öffnungsquerschnitt des Behälters eine bestimmte Wirkung im Wasser erzeugt. Dieser Öffnungsquerschnitt wird durch die entsprechende Markierung definiert.

Hinsichtlich der Dauer und Intensität der Reaktion kann es von Vorteil sein, wenn die Öffnung einen um die Achse X umlaufenden Umfang zwischen 15 mm und 55 mm oder einen Durchmesser von mindestens 3 mm und 12 mm aufweist. Insbesondere der Badezusatz in Tablettenform weist in diesem Fall eine Größe auf, die größer als die hier definierte Größe der Öffnung ist. Dadurch wird erreicht, dass die Tabletten beim Kippen des Behälters nicht aus der Öffnung herausfallen. Bevorzugt ist ein Umfang zwischen 28 mm und 38 mm oder ein Durchmesser von 6 mm vorgesehen.

Vorteilhaft kann es ferner sein, wenn der Badezusatz Carbonate und/oder Oxalate und/oder Amine und/oder Amide in Kombination mit einer Säure enthält und bei Kontakt mit Wasser unter der chemischen Reaktion ein Gas, bevorzugt CO₂ und/oder O₂ und/oder N₂, entsteht.

Außerdem kann es vorteilhaft sein, wenn der Badezusatz Duftstoffe und/oder Farben und/oder Pflegemittel enthält.

Ferner kann es vorteilhaft sein, wenn zwischen 45 g und 90 g oder zwischen 50 g und 70 g oder 64 g Badezusatz im System enthalten sind. Dadurch wird je nach Größe der Öffnung eine Reaktion zwischen 12 Minuten und 30 Minuten erreicht.

Dabei kann es von Vorteil sein, wenn die durchschnittliche Dichte des gesamten Systems in geschlossenem Zustand größerer 1,2 g/cm³ oder größer 1,0 g/cm³ ist. Durch die gegenüber Wasser größere Dichte des Systems, steht die Dosiervorrichtung auf dem Gefäßboden und schwimmt nicht auf. Die aufsteigenden Gase führen zu einem Sprudeln an der Wasseroberfläche und auch zur Schaumbildung 10. Würde die Dosiervorrichtung schwimmen, dann würde die Öffnung über der Wasseroberfläche stehen und das Gas würde direkt in die Luft abgegeben werden und es würde nicht sprudeln.

Behälter, die mit einem kugel- oder tablettenförmigen Badezusatz gefüllt sind und eine verschlossene Öffnung aufweisen, sind grundsätzlich im Stand der Technik bekannt. Diese Behälter dienen jedoch nicht als Dosiervorrichtung und werden gerade nicht zusammen mit dem Badezusatz in das Wasser gegeben. Vielmehr wird der Badezusatz nach dem Öffnen des Behälters in das Wasser gegeben und der Behälter für den nächsten Gebrauch wieder verschlossen oder nach dem Entleeren entsorgt. Hierzu ist die Öffnung am Behälter größer als die Formkörper des Badezusatzes, damit der Badezusatz aus dem Behälter ausgeschüttet werden kann. Der Vorteil der erfindungsgemäßen Verwendung liegt jedoch gerade darin, dass der Badezusatz sich im Behälter, der als Dosiervorrichtung dient, sprudelnd im Wasser auflöst, ohne sich im Badegefäß wie beispielsweise in der Badewanne zu verteilen.

Diesem speziellen System und der Verwendung liegt erfindungsgemäß ein Verfahren zum Dosieren eines in einem geschlossenen Behälter aufbewahrten Badezusatzes in Wasser zugrunde, das die folgenden Schritte aufweist: Mit dem Entfernen der Spitze des Behälters wird eine Öffnung erzeugt. Der Behälter wird danach zusammen mit dem Badezusatz in das Wasser eingetaucht, bis zumindest die Öffnung unter Wasser ist. Da der mit Badezusatz gefüllte Behälter nicht schwimmt, lässt man ihn einfach im Wasser untergehen. Nachdem sich nach einer gewissen Zeit ein Teil oder der gesamte Badezusatze in Verbindung mit Wasser sprudelnd aufgelöst hat, wird der Behälter aus dem Wasser genommen.

Es versteht sich von selbst, dass der jeweilige Formkörper des Badezusatzes beim Auflösen im Wasser immer kleiner wird und irgendwann ausreichend klein wird, um durch die Öffnung hindurch zu passen. Maßgeblich für die Erfindung ist jedoch das Stadium des Badezusatzes unmittelbar nach dem Öffnen des Behälters und während den ersten Minuten des Sprudelns.

Weitere Vorteile und Einzelheiten der Erfindung sind in den Patentansprüchen und in der Beschreibung erläutert und in den Figuren dargestellt. Es zeigt:
- Figur 1: ein Modell einer Dosiervorrichtung in Form eines Tetraeders;
- Figur 2: eine perspektivische Ansicht eines Behälters im geschlossenen Zustand;
- Figur 3a: eine perspektivische Ansicht eines Behälters ohne Verschlussteil;
- Figur 3b: eine perspektivische Ansicht eines Badezusatzes in Tablettenform gemäß Figur 3a;
- Figur 3c: eine Prinzipskizze einer Öffnung;
- Figur 4: eine Prinzipskizze der Geometrie der Behälterwand im Bereich einer Spitze;
- Figur 5: eine Prinzipskizze eines Systems unter Wasser.

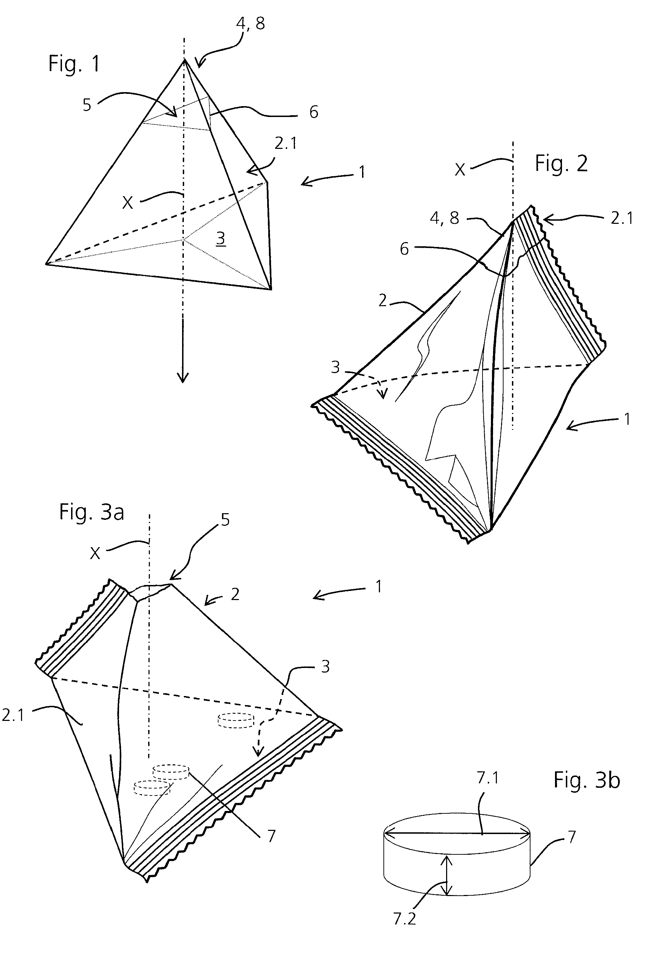

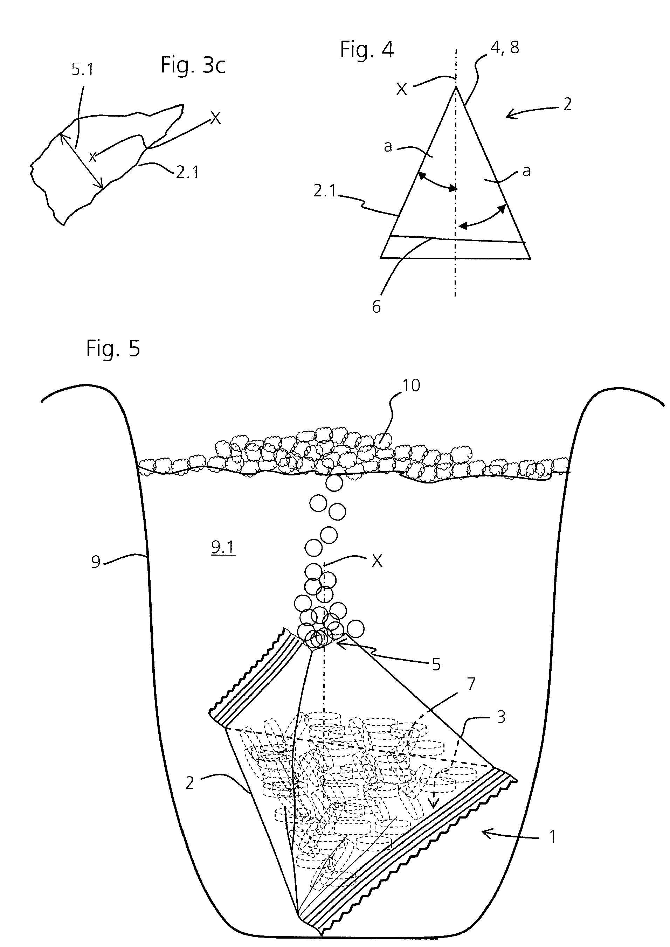

In Figur 1 ist eine Prinzipskizze und in Figur 2 ein Ausführungsbeispiel einer Dosiervorrichtung 1 dargestellt, die mit einem Badezusatz 7 (Figur 3a, 5) gefüllt als System zum Baden in das Wasser 9.1 gegeben wird. Die Dosiervorrichtung 1 ist aus einem Behälter 2 in Form eines Tetraeders gebildet. Der Tetraeder weist vier annähernd gleiche und dreieckförmige Flächen auf. Zu jeder Fläche gibt es jeweils eine nicht dargestellte Flächennormale N, die durch die jeweils gegenüberliegende Spitze 8 verläuft. Die gegenüber der oberen Spitze angeordnete Fläche bildet eine Standfläche 3. Die Spitze 8 bildet als ein Teil des Behälters 2 ein Verschlussteil 4. Hierzu wird die Spitze 8 entlang einer Markierung 6 von dem Behälter 2 abgetrennt. Mit dem Entfernen der Spitze 8 beziehungsweise des Verschlussteils 4 entsteht eine Öffnung 5, durch die Wasser 9.1 in die Dosiervorrichtung 1 eindringen kann. In der Dosiervorrichtung 1 ist ein in den Figuren 3a, 3b und 5 näher dargestellter Badezusatz 7 in Form von Tabletten enthalten. Der Badezusatz 7 reagiert mit dem in den Behälter 2 eingedrungenen Wasser 9.1 zu einem Gas. Das Gas steigt innerhalb der Dosiervorrichtung 1 nach oben und entweicht über die Öffnung 5.

Dadurch, dass die Dichte des Systems bestehend aus dem Behälter 2 und dem Badezusatz 7 größer ist als die Dichte von Wasser, steht die Dosiervorrichtung 1 bei der Anwendung in einem mit Wasser 9.1 gefüllten Badegefäß 9 wie in Figur 5 dargestellt, auf dem Gefäßboden. Das Gas strömt somit aus dem Behälter 2 in das Wasser 9.1 und steigt zur Wasseroberfläche. Dort entsteht neben einem aromatischen Geruch zusätzlich Badeschaum 10. Die im Badezusatz 7 enthaltenen löslichen Zusatzstoffe werden gleichzeitig im Badewasser 9.1 verteilt.

In Figur 3a ist eine Ansicht eines Systems einer Dosiervorrichtung 1 dargestellt, bei der der Behälter 2 beispielhaft mit drei Tabletten eines Badezusatzes 7 gefüllt ist. Die Tabletten bilden einen Formkörper. In einer nicht dargestellten Alternative ist der Badezusatz 7 als Granulat oder in Kugelform zu einem Formkörper geformt. In Figur 3b ist der Badezusatz 7 in Form einer Tablette vergrößert dargestellt. Die Tablette weist eine maximale und eine minimale Querschnittsfläche auf. Die minimale Querschnittsfläche wird in diesem Ausführungsbeispiel durch das Produkt des Durchmessers 7.1 und der Höhe 7.2 gebildet.

In Figur 3c ist die in Figur 3a dargestellte Öffnung 5 vergrößert dargestellt. Der Rand der Behälterwand 2.1 begrenzt die Querschittsfläche der Öffnung 5, die aufgrund der Elastizität des Materials der Behälterwand 2.1 variabel ist und eine mittlere Breite 5.1 aufweist. Erfindungsgemäß ist die größtmögliche Querschnittsfläche der Öffnung 5 kleiner als die minimalste Querschnittsfläche eines Formkörpers des Badezusatzes 7. Dadurch kann der Badezusatz 7 nach dem Öffnen des Behälters 2 nicht aus dem Behälter 2 ausgeschüttet werden. Hierzu trägt auch die in Figur 4 dargestellte Geometrie der Behälterwand 2.1 bei, die in Richtung der Achse X kegelförmig spitz zuläuft. Der Kegel beziehungsweise die Behälterwand 2.1 im Bereich der Spitze 8 ist in einem Winkel a von ungefhähr 22° [Grad] zur Achse X angeordnet. Dadurch wird verhindert, dass die Behälterwand 2.1 bei Kontakt mit Wasser 9.1 zusammenfällt und linienförmig aneinander haftet.

In Figur 5 ist die erfindungsgemäße Verwendung dargestellt, bei der das System bestehend aus dem Behälter 2 und dem Badezusatz 7 in einem Badegefäß 9 auf dem Grund aufliegt, das mit Wasser 9.1 gefüllt ist. Das durch die Reaktion mit Wasser erzeugte Gas tritt in Form von Blasen nach oben. Der Badezusatz 7 verteilt sich nicht im Wasser 9.1.

Wie in beiden Ausführungsbeispielen gemäß Figur 3a und 5 dargestellt, ist an dem Behälter 2 die Spitze 8 entlang der Markierung 6 entfernt. Der Behälter 2 ist geöffnet, wodurch Wasser 9.1 in der Behälter 2 eindringen kann. Im gleichen Moment entsteht durch die Reaktion von Wasser 9.1 mit dem Badezusatz 7 ein Gas, wie beispielsweise Kohlendioxid, das in dem Behälter 2 nach oben steigt und aus der Öffnung 5 austritt. Das ausströmende Gas verhindert, dass sich der Behälter 2 vollständig mit Wasser 9.1 füllt. Durch die Geometrie und die gegenüber Wasser 9.1 erhöhte Dichte steht das System automatisch auf dem Gefäßboden, auch wenn noch relativ viel Gas in dem Behälter 2 enthalten ist. Gegen Ende der Reaktion nimmt das Gewicht des Badezusatzes 7 ab und das System beginnt zu schwimmen.

Mit dem Gas entweicht auch Badeschaum 10, der bei der Reaktion von Badezusatz 7 mit Wasser 9.1 entsteht. Zusammen mit dem aufsteigenden Gas wird ein Sprudel-Schaumbad erzeugt. Durch die Dosiervorrichtung 1 verteilt sich der Badezusatz 7 nicht im Badegefäß 9, sodass auch der Nachteil von üblichen Badeperlen vermieden wird, die sich auf dem Gefäßboden Strukturen ähnlich wie Sand ablagern, auf denen man unangenehm sitzt.

Es versteht sich von selbst, dass der jeweilige Formkörper des Badezusatzes 7 beim Auflösen im Wasser 9.1 immer kleiner wird und irgendwann ausreichend klein wird, um durch die Öffnung 5 hindurch zu passen. Maßgeblich für die Erfindung ist jedoch das Stadium des Badezusatzes 7 unmittelbar nach dem Öffnen des Behälters 2 und während der ersten Minuten des Sprudelns.

## Patentansprüche

1. System bestehend aus einem als Formkörper gebildeten, kosmetischen Badezusatz (7) und einer Dosiervorrichtung (1), wobei der Badezusatz (7) in der Dosiervorrichtung (1) enthalten ist und bei Kontakt mit Wasser (9.1) unter Erzeugung eines Gases chemisch reagiert und die Dosiervorrichtung (1) durch einen Behälter (2) mit einer zumindest teilweise flexiblen Behälterwand (2.1) gebildet ist, wobei der Behälter (2) ein um eine Achse (X) angeordnetes und entfernbares Verschlussteil (4) für zumindest eine Öffnung (5) aufweist
**dadurch gekennzeichnet,**
**dass** die Größe des Formkörpers (7) relativ zu der Größe der Öffnung (5) so gewählt ist, dass der Formkörper (7) nicht durch die Öffnung (5) hindurch passt, wobei der Behälter (2) wasserunlöslich ist.

2. System nach Anspruch 1,
**dadurch gekennzeichnet,**
**dass** ein Ende des Behälters (2) in Richtung der Achse (X) eine Spitze (8) formt, wobei die Spitze (8) das Verschlussteil (4) bildet und durch das Entfernen der Spitze (8) die Öffnung (5) entsteht.

3. System nach Anspruch 2,
**dadurch gekennzeichnet,**
**dass** die Behälterwand (2.1) im Bereich der Spitze (8) um die Achse (X) umlaufend in einem Winkel (a) von mindestens 10° [Grad] relativ zur Achse (X) angeordnet ist.

4. System nach einem der vorhergehenden Ansprüche,
**dadurch gekennzeichnet,**
**dass** der Behälter (2) in Form eines Tetraeder ausgebildet eine rechtwinklig zu einer Achse (X) angeordnete Standfläche (3) aufweist und das Verschlussteil (4) in Richtung der Achse (X) gegenüberliegend zur Standfläche (3) angeordnet.

5. System nach einem der vorhergehenden Ansprüche 1 bis 4,
**dadurch gekennzeichnet,**
**dass** der Behälter (2) in Form eines Zylinders mit einem Verhältnis der Länge in Richtung der Achse (X) zum Durchmesser in einer Richtung rechtwinklig zur Achse (X) von mindestens einem Wert 10 ausgebildet ist.

6. System nach einem der vorhergehenden Ansprüche,
**dadurch gekennzeichnet,**
**dass** der Formkörper (7) in Tabletten- oder in Kugelform ausgebildet ist, wobei der mittlere Durchmesser der Tabletten oder der Kugeln zwischen 4 mm und 15 mm beträgt.

7. System nach einem der vorhergehenden Ansprüche,
**dadurch gekennzeichnet,**
**dass** der Behälter (2) aus einem Verbundstoff gebildet ist, der eine gas-und feuchtigkeitsdichte Innenschicht und eine auf die Innenschicht mittel-oder unmittelbar aufgebrachte Außenschicht aufweist, wobei die Außenschicht aus einem Trägermaterial und/oder aus einer Armierung gebildet ist.

8. System nach einem der vorhergehenden Ansprüche 2 bis 5,
**dadurch gekennzeichnet,**
**dass** der Behälter (2) um die Achse (X) umlaufend unterhalb der Spitze (4) eine Markierung (6), einen Schlitz und/oder eine Perforation aufweist, welche eine Ebene für die Öffnung (5) definiert.

9. System nach einem der vorhergehenden Ansprüche,
**dadurch gekennzeichnet,**
**dass** die Öffnung (5) einen um die Achse (X) umlaufenden Umfang zwischen 15 mm und 55 mm oder einen Durchmesser von mindestens 3 mm und 12 mm aufweist.

10. System nach einem der vorhergehenden Ansprüche,
**dadurch gekennzeichnet,**
**dass** der Badezusatz (7) Carbonate und/oder Oxalate und/oder Amine und/oder Amide in Kombination mit einer Säure enthält und bei Kontakt mit Wasser (9.1) unter der chemischen Reaktion ein Gas, bevorzugt CO₂ und/oder O₂ und/oder N₂, entsteht.

11. System nach einem der vorhergehenden Ansprüche,
**dadurch gekennzeichnet,**
**dass** zwischen 45 g und 90 g oder zwischen 50 g und 70 g oder 64 g Badezusatz (7) im System enthalten sind.

12. System nach einem der vorhergehenden Ansprüche,
**dadurch gekennzeichnet,**
**dass** die durchschnittliche Dichte des gesamten Systems bei verschlossener Dosiervorrichtung (1) größerer 1,2 g/cm³ oder größer 1,0 g/cm³ ist.

13. Verwendung eines Systems nach einem der vorstehenden Ansprüche zum Einbringen und Eintauchen in Wasser (9.1) und zum Erzeugen eines sprudelnden Gases.

14. Verfahren zum Dosieren eines in einem geschlossenen Behälter (2) aufbewahrten Badezusatzes (7) in Wasser (9.1), **gekennzeichnet durch** die folgenden Schritte:
1.) Erzeugen einer definierten Öffnung (5) am Behälter (2);
2.) Eintauchen des Behälters (2) mit dem Badezusatz (7) in Wasser (9.1), bis zumindest die Öffnung (5) unter Wasser (9.1) ist;
3.) Abwarten eines Teils oder der gesamten sprudelnden Reaktion des Badezusatzes (7) in Verbindung mit Wasser (9.1);
4.) Entnehmen des Behälters (2) aus dem Wasser (9.1).
